# EUROPEAN PATENT APPLICATION

(11) **EP 1 736 111 A2**
(43) Date of publication of application: **27.12.2006**
(21) Application number: 06252866.6
(22) Date of filing: 02.06.2006
(51) Int. Cl.: A61B 18/20, A61N 5/06

(54) **Dermatological treatment apparatus**

(30) Priority: 25.06.2005 GB 0513021
(71) Applicant: Lynton Lasers Ltd., Cheshire CW4 8AF (GB)
(72) Inventor: Berry, Andrew John, Cheadle, Cheshire SK8 7HN (GB); Charlton, Andrew, Morley Green, Cheshire SK9 5NX (GB)
(74) Representative: Hamilton, Alistair

(57) **Abstract**

A dermatological treatment platform is disclosed that incorporates an actively Q-switched laser and intense pulsed light source. The platform comprises: a power supply having an electrical energy source, a capacitive energy store for receiving energy from the energy source, a power outlet, and an electronically controlled switch that can selectively connect the energy store to the power outlet connection for delivery to a light source. The power supply further includes timing circuitry that can operate the electronically controlled switch and an optical switch within a device connected to the power supply. The capacitative energy source is charged in use to a potential suitable for operation of both the laser and the intense pulsed light source, typically less than 500V. Thus, a practitioner can offer both laser and intense pulsed light treatment without incurring the expense of two power supplies.

## Description

This invention relates to dermatological treatment apparatus. Specifically, it relates to a dermatological treatment platform incorporating an actively Q-switched laser and intense pulsed light that operates by application of high-intensity pulses of electromagnetic radiation in the visible or near-to-visible region with a very wide range of output parameters and applications.

Treatments in cosmetic dermatology typically work by a process of selective photothermolysis. This requires light of a specific wavelength to be absorbed by a target chromophore in the skin. The energy absorbed by the chromophore is converted to heat which can effectively destroy the target cells and thereby provide a cosmetic improvement.

In order to be effective, while keeping collateral tissue damage within reasonable limits, the wavelength and pulse duration must be selected carefully. Moreover, some treatments are performed using laser light while others are performed using high-intensity non-coherent light produced by a flashlamp.

The range of pulses used can range from 5ns to 500ms ― a factor in the order of 10⁸ with pulse energies ranging from 50mJ to 500J ― a factor in the order of 10⁴. For this reason, those working in the field have conventionally used two separate technologies to provide treatment apparatus that operates at opposite ends of this range. The short-pulse range has been provided by Q-switched lasers powered by supplies typically operating at voltages in excess of 1kV while the longer pulses have been provided by long-pulse lasers powered by electrically switched supplies typically operating at 500V or less. Intense pulse light units also typically operate in this lower voltage range. This is disadvantageous to practitioners, who must buy several treatment units if they are to offer a full range of treatments to their clients. This may be prohibitively expensive.

An aim of this invention is to provide dermatological treatment apparatus that is more versatile in its application than known apparatus.

Accordingly, from a first aspect this invention provides a dermatological treatment platform incorporating an actively Q-switched laser and intense pulsed light source comprising: a power supply having an electrical energy source, a capacitive energy store for receiving energy from the energy source, a power outlet, and an electronically controlled switch that can selectively connect the energy store to the power outlet connection for delivery to a light source; the power supply further including timing circuitry that can operate the electronically controlled switch and an optical switch within a device connected to the power supply, where the capacitative energy source is charged in use to a potential suitable for operation of both the laser and the intense pulsed light source.

Thus, the power supply can optionally operate to control the duration of a pulse produced by the light source either by controlling the time that the electronically controlled switch is closed, to operate a laser in long-pulse mode or an intense pulsed light, or it can control a laser pulse using the optical switch within a device connected to it.

Typically, the optical switch is a Q-switch. This may be embodied in a Pockels cell.

Typically, the electrical energy source delivers energy to the capacitive energy source to charge it to a potential not exceeding 500V. As is well known by those in the technical field, this voltage is suitable for powering intense pulsed lights and lasers in long-pulse mode. However, the present inventors have found that lasers can also be operated in Q-switched mode at this voltage.

Typically, a platform embodying the invention includes a plurality of light sources selected from a set including, an intense pulse light and a laser. In such embodiments, each light source can be readily connected to or disconnected from the power supply. In a typical configuration, the apparatus is embodied in an equipment case that contains the power supply and a laser light source, the intense pulsed light source being provided in an external handset that can be connected to the power supply for use. For example, there may be a connector provided upon the power supply to which the handset can be functionally connected. In such cases, light emitted from the laser light source is conducted to a treatment site through an articulated arm or a fibre delivery system.

The laser source may preferably be a neodymium-doped yttrium aluminium garnet (Nd:YAG) laser. Such lasers have a wide range of applications in the field of dermatology, but hitherto, that range has been available only to those prepared to obtain multiple power supplies.

Apparatus embodying the invention is typically capable of generating laser pulses of duration in the range 5ns to 100 ms, and may have an energy greater than 100 mJ per pulse. Most advantageously, the apparatus may be capable of producing laser pulses having the following combination of properties: a single pulse duration of less than 25 ns and pulse energy greater than 300 mJ, or a single pulse duration of greater than 1ms and pulse energy greater than 10 J.

Apparatus embodying the invention is also typically capable of generating intense pulse light pulses having a single pulse duration greater than 1ms and with an energy greater than 10J. In order to generate even longer pulses both the laser and the intense pulsed light are capable of delivering pulse trains with durations ranging from 1ms to 200ms. The intense pulsed light output energies that can be delivered to the skin may typically be in the range 10J - 250J (where 250J may be produced by 50J/cm² over an area of 5cm x 1cm from a treatment lightguide). Note that after losses due to wavelength filtering, inefficient collection into the lightguide of all light emitted and transmission losses through the various optical components and interfaces, substantially less than 100% of all light emitted by the flashlamp in the intense pulsed light system is actually delivered to the skin.

An embodiment of the invention will now be described in detail, by way of example, and with reference to the accompanying drawings, in which:

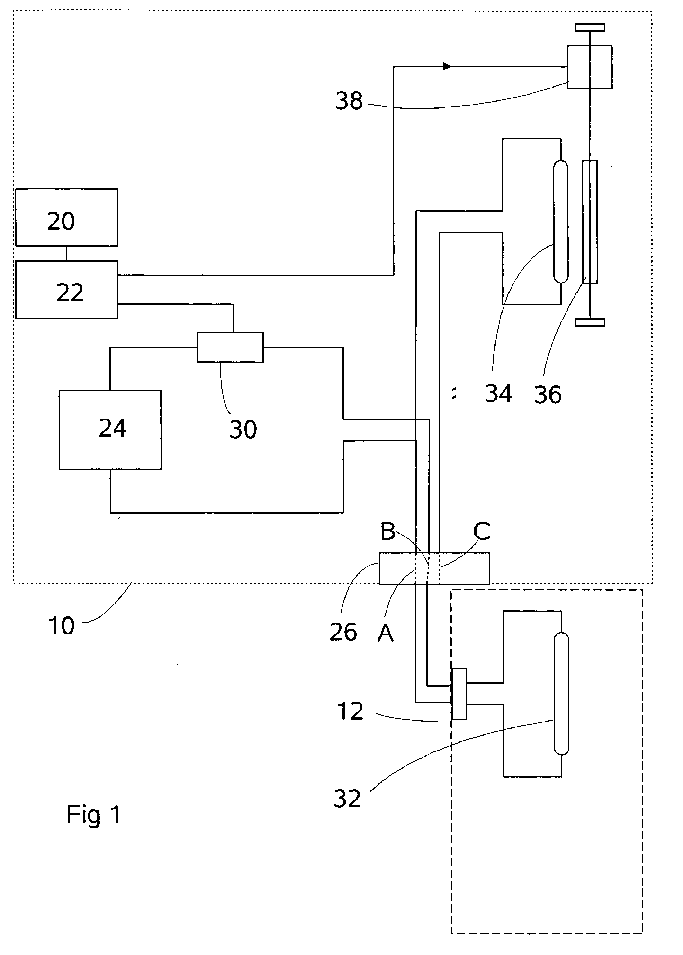

Figure 1 is a simplified block diagram of dermatological treatment apparatus embodying the invention.

Dermatological treatment apparatus comprises two main assemblies: a control and power unit 10, and a handset 12. The handset includes a cable having a connector that can be releasably connected to the control and power unit. The apparatus is typically provided as a set comprising one control and power unit and several handsets. The various handsets have different light sources to provide outputs having different characteristics to allow a practitioner to conduct different types of treatment. The output may be high-intensity non-coherent light from a flashlamp or it may be coherent laser light, typically produced using an Nd:YAG laser. In the latter case, the pulse duration may be in the order of several ns, or it may be a long pulse or pulse train, lasting up to 100 ms.

The control and power unit 10 comprises a control computer 20 that is responsible for controlling all aspects of the operation of the apparatus and for managing the task of interfacing with a user. In particular, the control computer is operative to control the operation of a high-precision timing circuit 22.

The control and power unit includes a power supply (not shown) that, in this embodiment, has an output of 500V DC (or less, in other embodiments) and is under the control of the control computer 20. This is connected to a capacitor bank 24 that acts to store energy from the power supply. The capacitor bank 24 is connected to an output connector 26 of the control and power unit 10. Between the capacitor bank 24 and the output connector 26 is an electronic switch, in this case being an insulated gate bipolar transistor (IGBT) 30. The IGBT can be switched by the timing circuit 22 to either connect the capacitor bank 24 to the connector 26 or to disconnect the capacitor bank 24 from the connector. In this way, the control computer 20, acting through the timing circuit 22, can control the delivery of electrical energy to a handset connected to the connector 26.

If a practitioner chooses to use intense pulsed light treatment, the treatment handset 12 is connected to the connector 26. Thus, electrical energy can be delivered to the handset by closure of the IGBT 30. On the other hand, if a practitioner chooses to use laser treatment, the treatment handset 12 is removed from the connector 26, and a coupling device (not shown) is inserted into the connector. This interconnects terminals B and C, as shown in the drawing, and insulates terminal A. Thus, electrical energy can be delivered to an exciting lamp 34 of the laser by closure of the IGBT 30.

To deliver short-pulse laser treatment, with pulse duration of less than 25ns, the laser is operated in Q-switched mode. Electrical energy is delivered to the excitation lamp 34 immediately upon closure of the IGBT 30, to pump the laser rod 36, and a Pockels cell Q-switch 38, at this time, is closed, so no light is emitted. The Q-switch 38 is opened under the control of the timing circuit 22 to generate an output pulse.

To deliver long-pulse laser treatment, with pulse or pulse train duration in the range of 1 ms to 100 ms, the laser is operated in long pulse mode. In this mode, the Q-switch 38 is open continuously, and the pulses are created by repeatedly opening and closing the IGBT 30 for periods corresponding to the required pulse length and period to deliver electrical pulses to the excitation lamp 34.

For intense pulsed light treatment, as described above, the supply of electrical energy is disconnected from the laser and applied to the flashlamp handset 12. In this mode, the pulses are created by repeatedly opening and closing the IGBT 30 for periods corresponding to the required pulse length and period, to deliver electrical pulses to the flashlamp 32.

Treatment apparatus for use with this embodiment can take one of three forms: short-pulse laser, long-pulse laser or intense pulsed light. In all cases, a practitioner can choose the pulse duration and pulse repetition rate appropriate to the type of treatment to be performed. In the case of the latter two types of treatment apparatus, this is achieved entirely by controlling the duration of the closure of the IGBT 30. While the IGBT 30 remains closed, electrical energy is delivered to an excitation light source 32 (in the case of a long-pulse laser) or the flashlamp. In contrast, in the case of a short-pulse laser, electrical energy is delivered to an excitation light source 34 immediately upon closure of the IGBT 30, to pump the laser rod 36. However, the pulse is not emitted until a Q-switch 38 is opened under the control of the timing circuit 22.

## Claims

1. A dermatological treatment platform incorporating an actively Q-switched laser and intense pulsed light source comprising: a power supply (10) having an electrical energy source, a capacitive energy store (24) for receiving energy from the energy source, a power outlet, and an electronically controlled switch (30) that can selectively connect the energy store to the power outlet connection for delivery to a light source; **characterised by** the power supply (10) further including timing circuitry (22) that can operate the electronically controlled switch and an optical switch (38) within a device connected to the power supply, where the capacitative energy source is charged in use to a potential suitable for operation of both the laser and the intense pulsed light source.

2. A dermatological treatment platform according to claim 1 in which the optical switch is a Q-switch.

3. A dermatological treatment platform according to claim 2 in which the Q-switch is embodied in a Pockels cell.

4. A dermatological treatment platform according to any preceding claim in which the electrical energy source delivers energy to the capacitive energy source to charge it to a potential not exceeding 500V.

5. A dermatological treatment platform according to any preceding claim having a plurality of light sources selected from a set including, but not limited to, an intense pulse light flashlamp (32) and an actively Q-switched laser (34, 36).

6. A dermatological treatment platform according to claim 5 in which each light source can be readily connected to or disconnected from the power supply.

7. A dermatological treatment platform according to claim 5 or claim 6 in which the apparatus is embodied in an equipment case that contains the power supply and a laser light source, a flashlamp being provided in an external handset that can be connected to the power supply for use.

8. A dermatological treatment platform according to claim 7 in which the laser light is transmitted to a treatment site through an articulated arm or a fibre delivery system.

9. A dermatological treatment platform according to any one of claims 5 to 8 in which there is a connector provided upon the power supply to which the handset or laser can be functionally connected.

10. A dermatological treatment platform according to any preceding claim having a neodymium-doped yttrium aluminium garnet (Nd:YAG) laser.

11. A dermatological treatment platform according to any preceding claim capable of generating laser pulses having the following combination of properties: a single pulse duration of less than 25 ns and pulse energy greater than 300 mJ, or a single pulse duration of greater than 1ms and pulse energy greater than 10 J.

12. A dermatological treatment platform according to any preceding claim capable of generating intense pulse light pulses having a pulse or pulse train duration in the range 1ms and 200ms, and pulse energy in the range 10J - 250J.
